(19) Europäisches Patentamt
European Patent Office
Office européen des brevets

(11) EP 3 318 572 B1

(12) EUROPEAN PATENT SPECIFICATION

(45) Date of publication and mention
of the grant of the patent:
**24.02.2021 Bulletin 2021/08**

(51) Int Cl.:
*C07K 5/12* *(2006.01)*    *A61P 43/00* *(2006.01)*
*A61P 13/12* *(2006.01)*    *A61P 9/12* *(2006.01)*
*A61P 9/00* *(2006.01)*    *A61K 38/05* *(2006.01)*
*A61K 36/8998* *(2006.01)*    *A61K 36/82* *(2006.01)*
*A61K 36/48* *(2006.01)*    *A61K 31/405* *(2006.01)*
*A61K 31/198* *(2006.01)*    *A61K 38/12* *(2006.01)*

(21) Application number: **16818003.2**

(22) Date of filing: **30.06.2016**

(86) International application number:
**PCT/JP2016/069381**

(87) International publication number:
**WO 2017/002895 (05.01.2017 Gazette 2017/01)**

(54) **COMPOSITION COMPRISING AMINO ACID AND CYCLIC DIPEPTIDE**

ZUSAMMENSETZUNG UMFASSEND AMINOSÄURE UND CYCLISCHES DIPEPTID

COMPOSITION COMPRENANT UN ACIDE AMINÉ ET UN DIPEPTIDE CYCLIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.07.2015 JP 2015132688**

(43) Date of publication of application:
**09.05.2018 Bulletin 2018/19**

(73) Proprietor: **Suntory Holdings Limited
Osaka-shi, Osaka 530-8203 (JP)**

(72) Inventors:
• **FUKIZAWA, Shinya
Soraku-gun
Kyoto 619-0284 (JP)**
• **YAMAMOTO, Kenji
Soraku-gun
Kyoto 619-0284 (JP)**
• **SAITO, Masayuki
Soraku-gun
Kyoto 619-0284 (JP)**

(74) Representative: **Vossius & Partner
Patentanwälte Rechtsanwälte mbB
Siebertstrasse 3
81675 München (DE)**

(56) References cited:
WO-A1-2005/063245    JP-A- S5 973 574
JP-A- 2005 206 528    JP-A- 2008 542 307

• KEIKO UEDA: 'Amino-san, Oligopeptide Oyobi
Shokuhin no Hito Kessei ACE Sogai Sayo ni
Tsuite' THE JAPANESE SOCIETY OF NUTRITION
AND FOOD SCIENCE TAIKAI KOEN YOSHISHU
vol. 61, 2007, page 123, XP009508034
• PRASAD, C.: 'Bioactive cyclic dipeptides'
PEPTIDES vol. 16, no. 1, 1995, ISSN 0196-9781
pages 151 - 164, XP002477201

**Description**

TECHNICAL FIELD

The present invention relates to a composition containing an amino acid and a cyclic dipeptide. More specifically, the present invention relates to a composition containing an amino acid or a salt thereof and a cyclic dipeptide or a salt thereof, wherein the ratio of the total amount of the amino acid or salt thereof to the total amount of the cyclic dipeptide or salt thereof falls within a specific range, use of the composition for inhibiting angiotensin-converting enzyme, and a method for inhibiting angiotensin-converting enzyme.

BACKGROUND ART

It is widely known that lifestyle-related diseases such as hypertension or hyperlipidemia are caused by change in eating habits, a lack of exercise, stress, smoking, genetic factors, etc. Particularly, hypertension is characterized in that its symptoms progress without being subjectively noticed, and exhibits a very high morbidity, also including hypertension suspects. Also, hypertension is known to lead to serious diseases such as stroke (cerebral infarction, cerebral hemorrhage, subarachnoid hemorrhage, etc.), heart disease (angina pectoris, myocardial infarction, cardiomegaly, heart failure, etc.), and renal failure (hypertensive nephropathy, etc.). Therefore, there is a social demand for prevention and early amelioration of hypertension.

As one of the solutions of hypertension, it is known that inhibition of angiotensin-converting enzyme (also called angiotensin I-converting enzyme) in the renin-angiotensin system known as a mechanism to elevate blood pressure is effective. The angiotensin-converting enzyme is mainly present in the lung, vascular endothelial cells, and the renal proximal tubule and has the property of converting the substrate angiotensin I to angiotensin II serving as a pressor hormone. The angiotensin-converting enzyme also has an action of decomposing bradykinin having a vasodilating action, in addition to the action of yielding angiotensin II. Therefore, angiotensin-converting enzyme inhibitors can also exert a blood pressure-lowering action through the mechanism of a bradykinin decomposition-inhibiting action. Thus, the angiotensin-converting enzyme inhibitors are clinically effective for treatment or prevention of hypertension.

Against this backdrop, amino acids have been found to have a blood pressure-lowering activity or an angiotensin-converting enzyme-inhibiting activity. For example, NPL 1 discloses that decrease in mean arterial pressure is observed by the oral administration of histidine to spontaneously hypertensive rats. However, it is difficult to achieve a sufficient blood pressure-lowering activity by the angiotensin-converting enzyme-inhibiting activity brought about by an amino acid alone.

NPL 2 discloses that a Maillard reaction product obtained from the combination of a specific amino acid and glucose or xylose has an angiotensin-converting enzyme-inhibiting activity. However, a Maillard reaction product, such as acrylamide, formed through the reaction of asparagine with the reducing sugar is suspected of having biological toxicity.

It has been further reported that a composition containing a linear dipeptide or tripeptide including proline, and a free amino acid exhibits an angiotensin-converting enzyme-inhibiting activity (PTL 1). However, the linear dipeptide exposes an amino group and a carboxyl group, which are polar groups, at its ends and is therefore disadvantageous that it has low lipid solubility and is less bioabsorbable.

CITATION LIST

PATENT LITERATURE

PTL 1: Japanese Patent Laid-Open No. 2011-102327

NON PATENT LITERATURE

    NPL 1: Clin Exp Pharmacol Physiol., 37(1), 62-68(2010)
    NPL 2: J. Brew. Soc. Japan., 96(3), 199-206(2001)

SUMMARY OF INVENTION

TECHNICAL PROBLEM

An object of the present invention is, for example, to provide a composition which has high bioabsorbability and excellent biological safety and can enhance an angiotensin-converting enzyme-inhibiting activity ascribable to an amino acid.

SOLUTION TO PROBLEM

The present inventors have conducted diligent studies on the object and consequently focused on utilization of cyclic dipeptides or salts thereof. Cyclic dipeptides are dipeptides having a cyclic structure generated through dehydration condensation of an amino group and a carboxyl group each present in the end of a linear dipeptide. In recent years, their various physiological activities have received attention. The present inventors have found that the ratio of the total amount of an amino acid or a salt thereof to the total amount of a cyclic dipeptide or a salt thereof in a composition is set to within a specific range to thereby enhance an angiotens in-converting enzyme-inhibiting activity brought about by the amino acid alone, and arrived at the completion of the present invention.

Specifically, the present invention relates to, but is not limited to, the following aspects:

(1) A composition comprising an amino acid or a salt thereof and a cyclic dipeptide or a salt thereof, wherein
the amino acid is one or two or more members selected from the group consisting of glycine, alanine, valine, leucine, isoleucine, serine, threonine, aspartic acid, glutamic acid, asparagine, glutamine, arginine, lysine, cysteine, methionine, phenylalanine, tyrosine, tryptophan, histidine, proline, and hydroxyproline,
the cyclic dipeptide is one or two or more members selected from the group consisting of cyclo-glutamyl-histidine [Cyclo(Glu-His)], cyclo-arginyl-tyrosine [Cyclo(Arg-Tyr)], and cyclo-isoleucyl-serine [Cyclo(Ile-Ser)], and
the ratio of the total amount of the amino acid or salt thereof to the total amount of the cyclic dipeptide or salt thereof is 1:0.02 to 1:20.
(2) The composition according to (1), wherein
the amino acid is leucine, and
the ratio of the total amount of the amino acid or salt thereof to the total amount of the cyclic dipeptide or salt thereof is 1:0.02 to 1:1.5.
(3) The composition according to (1), wherein
the amino acid is threonine, and
the ratio of the total amount of the amino acid or salt thereof to the total amount of the cyclic dipeptide or salt thereof is 1:0.02 to 1:1.5.
(4) The composition according to (1), wherein
the amino acid is arginine, and
the ratio of the total amount of the amino acid or salt thereof to the total amount of the cyclic dipeptide or salt thereof is 1:0.02 to 1:1.5.
(5) The composition according to (1), wherein
the amino acid is tyrosine, and
the ratio of the total amount of the amino acid or salt thereof to the total amount of the cyclic dipeptide or salt thereof is 1:0.10 to 1:1.5.
(6) The composition according to (1), wherein
the amino acid is tryptophan, and
the ratio of the total amount of the amino acid or salt thereof to the total amount of the cyclic dipeptide or salt thereof is 1:0.02 to 1:1.5.
(7) The composition according to any one of (1) to (6), wherein the total amount of the cyclic dipeptide or salt thereof is 1.0 ppm/Brix to $1.0 \times 10^4$ ppm/Brix.
(8) The composition according to any one of (1) to (6), wherein the total amount of the cyclic dipeptide or salt thereof is 5.0 ppm/Brix to $1.0 \times 10^4$ ppm/Brix.
(9) The composition according to any one of (1) to (8), wherein the amino acid or salt thereof is obtained from a soybean peptide, a tea peptide, or a malt peptide.
(10) The composition according to any one of (1) to (9), wherein the cyclic dipeptide or salt thereof is obtained from a soybean peptide, a tea peptide, or a malt peptide.
(11) The composition according to any one of (1) to (10), wherein the composition has an angiotensin-converting enzyme-inhibiting action.
(12) The composition according to any one of (1) to (11), wherein the composition is for suppression of elevation of blood pressure, for lowering of blood pressure, for protection of renal functions, or for prevention or amelioration of stroke or heart disease.
(13) The composition according to any one of (1) to (12), wherein the composition is provided with a label indicating a function to be exerted through inhibition of angiotensin-converting enzyme.
(14) The composition according to (13), wherein the functional indication is selected from the group consisting of "expecting lowering of blood pressure", "suppressing elevation of blood pressure", "slowing elevation of blood pressure", "suppressing rapid elevation of blood pressure upon awakening", "preventing hypertension", "helping ameliorating hypertension", "protecting renal functions", and "ameliorating renal functions".

(15) A composition comprising an amino acid or a salt thereof and a cyclic dipeptide or a salt thereof for use in inhibiting angiotensin-converting enzyme, wherein the amino acid is one or two or more members selected from the group consisting of glycine, alanine, valine, leucine, isoleucine, serine, threonine, aspartic acid, glutamic acid, asparagine, glutamine, arginine, lysine, cysteine, methionine, phenylalanine, tyrosine, tryptophan, histidine, proline, and hydroxyproline,

the cyclic dipeptide is one or two or more members selected from the group consisting of cyclo-glutamyl-histidine [Cyclo(Glu-His)], cyclo-arginyl-tyrosine [Cyclo(Arg-Tyr)], and cyclo-isoleucyl-serine [Cyclo(Ile-Ser)], and

the ratio of the total amount of the amino acid or salt thereof to the total amount of the cyclic dipeptide or salt thereof is 1:0.02 to 1:20.

(16) The composition for use according to (15), wherein the amino acid includes one or two or more members selected from the group consisting of leucine, threonine, arginine, tyrosine, and tryptophan.

(17) A composition for use in a method for inhibiting angiotensin-converting enzyme using a composition comprising an amino acid or a salt thereof and a cyclic dipeptide or a salt thereof, wherein

the amino acid is one or two or more members selected from the group consisting of glycine, alanine, valine, leucine, isoleucine, serine, threonine, aspartic acid, glutamic acid, asparagine, glutamine, arginine, lysine, cysteine, methionine, phenylalanine, tyrosine, tryptophan, histidine, proline, and hydroxyproline,

the cyclic dipeptide is one or two or more members selected from the group consisting of cyclo-glutamyl-histidine [Cyclo(Glu-His)], cyclo-arginyl-tyrosine [Cyclo(Arg-Tyr)], and cyclo-isoleucyl-serine [Cyclo(Ile-Ser)], and

the ratio of the total amount of the amino acid or salt thereof to the total amount of the cyclic dipeptide or salt thereof is 1:0.02 to 1:20.

(18) The composition for use in a method according to (17), wherein the amino acid includes one or two or more members selected from the group consisting of leucine, threonine, arginine, tyrosine, and tryptophan.

ADVANTAGEOUS EFFECTS OF INVENTION

The present invention can provide a composition which enhances an angiotensin-converting enzyme-inhibiting activity brought about by an amino acid alone . A blood pressure elevation-suppressing action or a blood pressure-lowering action is obtained through inhibition of angiotensin-converting enzyme. Hence, a hypertension-preventing or -ameliorating effect or a preventing or ameliorating effect on various diseases caused by hypertension, such as stroke, heart disease, and renal failure, is exerted by ingestion of the composition according to the present invention.

The amino acid contained in the composition according to the present invention is a naturally derived component, and the cyclic dipeptide is also contained in a heat-treated product of a plant-derived peptide, or the like. Thus, these components are highly safe. Therefore, the composition according to the present invention is considered to have a very low risk of manifesting serious side effects. Cyclic dipeptides have higher lipid solubility than that of linear dipeptides and are not mere dipeptides composed of only a peptide linkage. Thus, the cyclic dipeptide or salt thereof contained in the composition according to the present invention has resistance to the actions of various peptidases secreted into the gastrointestinal tract and can also be expected to have high gastrointestinal absorbability.

DESCRIPTION OF EMBODIMENTS

1. Amino acid or salt thereof

Throughout the present specification, "amino acid" means a compound having both of an amino group and a carboxyl group as functional groups. Amino acids in the present specification may be essential amino acids or non-essential amino acids or may be amino acids formed through modification after protein synthesis. Throughout the present specification, "amino acid or salt thereof" is occasionally referred to as "amino acid" in a collective and simple manner.

The amino acid contained in the composition according to the present invention is one or two or more members selected from the group consisting of glycine, alanine, valine, leucine, isoleucine, serine, threonine, aspartic acid, glutamic acid, asparagine, glutamine, arginine, lysine, cysteine, methionine, phenylalanine, tyrosine, tryptophan, histidine, proline, and hydroxyproline. More preferably, three or more members selected from these amino acids are used as an active ingredient. Among the amino acids described above or salts thereof, one or two or more members selected from the group consisting of leucine, threonine, arginine, tyrosine, and tryptophan are preferred, and three or more members selected from these amino acids or salts thereof are more preferably contained.

Throughout the present specification, "salt of an amino acid" refers to any pharmacologically acceptable salt (including inorganic salts and organic salts) of the amino acid. Examples thereof include, but not limited to, sodium salts, potassium salts, calcium salts, magnesium salts, ammonium salts, hydrochlorides, sulfates, nitrates, phosphates, and organic acid salts (acetates, citrates, maleates, malates, oxalates, lactates, succinates, fumarates, propionates, formates, benzoates, picrates, benzenesulfonates, trifluoroacetates, etc.) of the amino acid.

Amino acids or salts thereof used in the present invention may be self-made by using a known method, or commercially available products may be used. For example, an amino acid or a salt thereof can be produced by chemical synthesis, extraction from a natural product, an enzymatic method, or microbial fermentation. For example, a treatment product obtained by subjecting a plant-derived peptide, for example, a soybean peptide, a tea peptide, or a malt peptide, to enzyme treatment or the like can be suitably used. Alternatively, an amino acid or a salt thereof contained in an existing composition may be used as the amino acid or salt thereof according to the present invention. If the content of a specific amino acid or a salt thereof in an existing composition is less than the desired content, the shortfall of the specific amino acid or salt thereof may be appropriately supplied by using a commercially available product, a synthesized product, or the like.

In the present invention, the amino acid or salt thereof may be obtained from a plant-derived peptide or may be obtained from a heat-treated product of a plant-derived peptide.

## 2. Cyclic dipeptide or salt thereof

Throughout the present specification, "cyclic dipeptide" refers to a cyclic dipeptide including amino acids as constituent units and having a diketopiperazine structure generated through dehydration condensation of an amino group of an amino acid and a carboxyl group of another amino acid. Throughout the present specification, "cyclic dipeptide or salt thereof" is occasionally referred to as "cyclic dipeptide" in a collective and simple manner. Throughout the present specification, the order of amino acids in a cyclic dipeptide may be inverse as long as the constitution is unchanged, and, for example, Cyclo(Glu-His) and Cyclo(His-Glu) represent an identical cyclic dipeptide.

In a cyclic dipeptide, end portions of two amino acids bond together via an amide linkage (i.e., a cyclic dipeptide has a cyclic structure formed by the amino linkage between an amino end and a carboxy end), and thus cyclic dipeptides are characterized by higher lipid solubility than that of linear dipeptides (particularly, linear dipeptides consisting of the same type of amino acid composition), in which a carboxyl group and an amino group as polar groups are exposed at molecular end portions. Accordingly, cyclic dipeptides are suprior in gastrointestinal permeability and membrane permeability to linear dipeptides. This is also evident from previously reported results of compound permeation tests using everted rat gut sacs (J. Pharmacol, 1998, 50: 167-172). Also, cyclic dipeptides are considered to also have higher resistance to various peptidases because of their specific structures.

The cyclic dipeptide or salt thereof included in the present invention is one or two or more members selected from the group consisting of cyclo-glutamyl-histidine [Cyclo(Glu-His)], cyclo-arginyl-tyrosine [Cyclo(Arg-Tyr)], and cyclo-isoleucyl-serine [Cyclo(Ile-Ser)]. More preferably, all of these cyclic dipeptides or salts thereof are contained.

Throughout the present specification, "salt of a cyclic dipeptide" refers to any pharmacologically acceptable salt (including inorganic salts and organic salts) of the cyclic dipeptide. Examples thereof include, but not limited to, sodiumsalts, potassium salts, calcium salts, magnesium salts, ammonium salts, hydrochlorides, sulfates, nitrates, phosphates, and organic acid salts (acetates, citrates, maleates, malates, oxalates, lactates, succinates, fumarates, propionates, formates, benzoates, picrages, benzenesulfonates, trifluoroacetates, etc.) of the cyclic dipeptide. Those skilled in the art can easily prepare a salt of a cyclic dipeptide by using any method known in the art.

Cyclic dipeptides used in the present invention can be prepared in accordance with a method known in the art. For example, a cyclic dipeptide may be produced by using chemical synthesis, an enzymatic method, or microbial fermentation, or may be synthesized by using dehydration and cyclization of a linear peptide, or may be prepared in accordance with a method described in Japanese Patent Laid-Open No. 2003-252896, Journal of Peptide Science, 10, 737-737, 2004, International Publication No. WO 2014/200000, etc. For example, a heat-treated product of a plant-derived peptide rich in cyclic dipeptides can be obtained by subjecting a plant-derived peptide obtained through enzyme treatment or heat treatment of a raw material including a plant-derived protein to further high-temperature heat treatment. As desired, the heat-treated product of the plant-derived peptide obtained may be subjected to a process such as filtration, centrifugation, concentration, ultrafiltration, freeze-drying, and pulverization. If the content of a specific cyclic dipeptide in a heat-treated product of a plant-derived peptide is less than the desired content, the shortage of the specific cyclic dipeptide may be appropriately supplied by using other plant-derived peptides, a commercially available product, or a synthesized product.

In the present invention, the cyclic dipeptide or salt thereof may be obtained from a plant-derived peptide or may be obtained from a heat-treated product of a plant-derived peptide.

## 3. Plant-derived peptide

Throughout the present specification, "plant-derived peptide" is not particularly limited, and, for example, a soybean peptide, a tea peptide, or a malt peptide can be used. A plant-derived peptide may be prepared from a plant-derived protein or a raw material including the protein, or a commercially available product may be used.

### 3-1. Soybean peptide

Throughout the present specification, "soybean peptide" refers to a low-molecular peptide obtained by lowering the molecular weight of a protein through enzyme treatment or heat treatment of a soybean protein. The soybean (scientific name: *Glycine max*) as the raw material may be any species and may be produced in any area. Soybean in a stage of processing, such as crushed soybean, can also be used.

### 3-2. Tea peptide

Throughout the present specification, "tea peptide" refers to a tea-derived low-molecular peptide obtained by lowering the molecular weight of a protein through enzyme treatment or heat treatment of a tea (including tea leaves and used tea leaves) extract. The tea leaves of the extraction raw material are drinkable parts by extraction of a tea plant (scientific name: *Camellia sinensis*), such as leaves and stems of tea leaves. In addition, the tea leaves may be in any form, such as a macrophyll or powder form. The harvest time of tea leaves may be any time and is appropriately selected to obtain a desired flavor.

### 3-3. Malt peptide

Through the present specification, "malt peptide" refers to a malt-derived low-molecular peptide obtained by lowering the molecular weight of a protein through enzyme treatment or heat treatment of an extract obtained from malt or a crushed product thereof. The malt peptide as the raw material may be any species and may be produced in any area. In particular, barley malt, which is germinated seeds of barley, is suitably used. Throughout the present specification, barley malt is also referred to as "malt" in a simple manner.

### 4. Composition

One aspect of the present invention is a composition containing an amino acid or a salt thereof and a cyclic dipeptide or a salt thereof, wherein the ratio of the total amount of the amino acid or salt thereof to the total amount of the cyclic dipeptide or salt thereof falls within a specific range.

For the present invention, it is important that the ratio of the total amount of the amino acid or salt thereof to the total amount of the cyclic dipeptide or salt thereof in the composition should fall within a specific range. Specifically, the ratio of the total amount of the amino acid or salt thereof to the total amount of the cyclic dipeptide or salt thereof in the composition (the total amount of the amino acid or salt thereof in the composition : the total amount of the cyclic dipeptide or salt thereof in the composition) is 1:0.02 or more, preferably 1:0.05 or more, more preferably 1:0.1 or more, and 1:20 or less, preferably 1:10 or less, 1:5 or less, 1:2 or less, 1:1.5 or less, or 1:0.5 or less. Typically, the range of the ratio of the total amount of the amino acid or salt thereof to the total amount of the cyclic dipeptide or salt thereof in the composition is 1: 0.02 to 1: 20, preferably 1:0.02 to 1:5, 1:0.02 to 1:2, more preferably 1:0.02 to 1:1.5.

The amino acid or salt thereof contained in the present invention is one or two or more members selected from the group consisting of glycine, alanine, valine, leucine, isoleucine, serine, threonine, aspartic acid, glutamic acid, asparagine, glutamine, arginine, lysine, cysteine, methionine, phenylalanine, tyrosine, tryptophan, histidine, proline, and hydroxyproline. Preferably, three or more members selected from these amino acids or salts thereof are contained.

Among the amino acids described above or salts thereof, one or two or more members selected from the group consisting of leucine, threonine, arginine, tyrosine, and tryptophan are preferred, and three or more members selected from these amino acids or salts thereof are more preferably contained.

The ratio of the total amount of the leucine or salt thereof to the total amount of the cyclic dipeptide or salt thereof in the composition (the total amount of the leucine or salt thereof in the composition : the total amount of the cyclic dipeptide or salt thereof in the composition) is preferably 1:0.02 to 1:1.5 or 1:0.05 to 1:1.5, more preferably 1:0.10 to 1:1.5, still more preferably 1:0.10 to 1:0.50.

The ratio of the total amount of the threonine or salt thereof to the total amount of the cyclic dipeptide or salt thereof in the composition (the total amount of the threonine or salt thereof in the composition : the total amount of the cyclic dipeptide or salt thereof in the composition) is preferably 1:0.02 to 1:1.5, more preferably 1:0.10 to 1:1.5, still more preferably 1:0.10 to 1:0.50.

The ratio of the total amount of the arginine or salt thereof to the total amount of the cyclic dipeptide or salt thereof in the composition (the total amount of the arginine or salt thereof in the composition : the total amount of the cyclic dipeptide or salt thereof in the composition) is preferably 1:0.02 to 1:1.5, more preferably 1:0.10 to 1:1.5, still more preferably 1:0.10 to 1:0.50.

The ratio of the total amount of the tyrosine or salt thereof to the total amount of the cyclic dipeptide or salt thereof in the composition (the total amount of the tyrosine or salt thereof in the composition : the total amount of the cyclic dipeptide or salt thereof in the composition) is preferably 1:0.10 to 1:1.5, more preferably 1:0.10 to 1:0.50.

The ratio of the total amount of the tryptophan or salt thereof to the total amount of the cyclic dipeptide or salt thereof in the composition (the total amount of the tryptophan or salt thereof in the composition : the total amount of the cyclic dipeptide or salt thereof in the composition) is preferably 1:0.02 to 1:1.5 or 1:0.05 to 1:1.5, more preferably 1:0.10 to 1:1.5, still more preferably 1:0.10 to 1:0.50.

The cyclic dipeptide or salt thereof included in the present invention is one or two or more members selected from the group consisting of cyclo-glutamyl-histidine [Cyclo(Glu-His)], cyclo-arginyl-tyrosine [Cyclo(Arg-Tyr)], and cyclo-isoleucyl-serine [Cyclo(Ile-Ser)]. Preferably, three or more selected from these cyclic dipeptides or salts thereof are contained.

The total amount of the amino acid or salt thereof in the composition according to the present invention is not particularly limited as long as the ratio of the total amount of the amino acid or salt thereof to the total amount of the cyclic dipeptide or salt thereof in the composition satisfies the range described above, in view of form of administration, an administration method, etc. For example, in the case of using a plant-derived peptide such as a soybean peptide, a tea peptide, or a malt peptide as a raw material, the total amount of the amino acid or salt thereof in the composition according to the present invention is, in terms of a content per Brix, 1.0 ppm/Brix or more, preferably 10 ppm/Brix or more, more preferably $1.0 \times 10^2$ ppm/Brix or more, and $1.0 \times 10^4$ ppm/Brix or less, preferably $5.0 \times 10^3$ ppm/Brix or less, more preferably $1.0 \times 10^3$ ppm/Brix or less, and is typically, 1.0 ppm/Brix to $1.0 \times 10^4$ ppm/Brix, preferably 10 ppm/Brix to $5.0 \times 10^3$ ppm/Brix, more preferably $1.0 \times 10^2$ ppm/Brix to $1.0 \times 10^3$ ppm/Brix. In the case of using a plant-derived peptide such as a soybean peptide, a tea peptide, or a malt peptide as a raw material, the individual content of glycine, alanine, valine, leucine, isoleucine, serine, threonine, aspartic acid, glutamic acid, asparagine, glutamine, arginine, lysine, cysteine, methionine, phenylalanine, tyrosine, tryptophan, histidine, proline, hydroxyproline, or each corresponding salt in the composition according to the present invention is, in terms of a content per Brix, 0.1 ppm/Brix or more, preferably 1.0 ppm/Brix or more, more preferably 5.0 ppm/Brix or more, and $1.0 \times 10^3$ ppm/Brix or less, preferably $5.0 \times 10^2$ ppm/Brix or less, more preferably $1.0 \times 10^2$ ppm/Brix or less, and is typically 0.1 ppm/Brix to $1.0 \times 10^3$ ppm/Brix, preferably 1.0 ppm/Brix to $5.0 \times 10^2$ ppm/Brix, more preferably 5.0 ppm/Brix to $1.0 \times 10^2$ ppm/Brix.

In the present invention, the content of an amino acid or a salt thereof can be indicated by an amount per Brix as described above. Throughout the present specification, "amount per Brix" means an amount defined by a value corresponding to mass percentage of a sucrose solution (an aqueous solution containing only sucrose as a solute) of 20°C. "ppm" used in the present specification refers to ppm in weight / volume (w/v), unless otherwise specified, and 1.0 ppm/Brix can be converted into 0.1 mg/mL or 0.01% by weight when the specific gravity of the solvent is 1.

The content of the amino acid or salt thereof added to the composition according to the present invention can be measured in accordance with a known method. The content can be measured by using, for example, LC-MS/MS or a saccharimeter.

In the case of using a synthesized product or a purified product as the amino acid or salt thereof, the total content of amino acids or salts thereof in the composition according to the present invention is not particularly limited and is, for example, in terms of a content per Brix, 1.0 ppm/Brix or more, preferably 10 ppm/Brix or more, more preferably $1.0 \times 10^2$ ppm/Brix or more, and $1.0 \times 10^4$ ppm/Brix or less, preferably $5.0 \times 10^3$ ppm/Brix or less, more preferably $1.0 \times 10^3$ ppm/Brix or less, and is typically 1.0 ppm/Brix to $1.0 \times 10^4$ ppm/Brix, preferably 10 ppm/Brix to $5.0 \times 10^3$ ppm/Brix, more preferably $1.0 \times 10^2$ ppm/Brix to $1.0 \times 10^3$ ppm/Brix. In the case of using a synthesized product or a purified product as the amino acid or salt thereof, the individual content of glycine, alanine, valine, leucine, isoleucine, serine, threonine, aspartic acid, glutamic acid, asparagine, glutamine, arginine, lysine, cysteine, methionine, phenylalanine, tyrosine, tryptophan, histidine, proline, hydroxyproline, or each corresponding salt in the composition according to the present invention is, in terms of a content per Brix, 0.1 ppm/Brix or more, preferably 1.0 ppm/Brix or more, more preferably 5.0 ppm/Brix or more, and $1.0 \times 10^3$ ppm/Brix or less, preferably $5.0 \times 10^2$ ppm/Brix or less, more preferably $1.0 \times 10^2$ ppm/Brix or less, and is typically 0.1 ppm/Brix to $1.0 \times 10^3$ ppm/Brix, preferably 1.0 ppm/Brix to $5.0 \times 10^2$ ppm/Brix, more preferably 5.0 ppm/Brix to $1.0 \times 10^2$ ppm/Brix.

The total amount of the cyclic dipeptide or salt thereof in the composition according to the present invention is not particularly limited as long as the ratio of the total amount of the amino acid or salt thereof in the composition satisfies the range described above, in view of form of administration, an administration method, etc. For example, in the case of using a plant-derived peptide as a raw material, the total amount of cyclo-glutamyl-histidine [Cyclo(Glu-His)], cyclo-arginyl-tyrosine [Cyclo(Arg-Tyr)], and cyclo-isoleucyl-serine [Cyclo(Ile-Ser)], and their corresponding salts in the composition according to the present invention is, in terms of a content per Brix, 1.0 ppm/Brix or more, preferably 5.0 ppm/Brix or more, more preferably 10 ppm/Brix or more, and $1.0 \times 10^4$ ppm/Brix or less, preferably $5.0 \times 10^3$ ppm/Brix or less, more preferably $1.0 \times 10^3$ ppm/Brix or less, and is typically 1.0 ppm/Brix to $1.0 \times 10^4$ ppm/Brix, preferably 5.0 ppm/Brix to $1.0 \times 10^4$ ppm/Brix, more preferably 5.0 ppm/Brix to $5.0 \times 10^3$ ppm/Brix, still more preferably 10 ppm/Brix to $1.0 \times$

$10^3$ ppm/Brix. In the case of using a plant-derived peptide as a raw material, the individual content of cyclo-glutamyl-histidine [Cyclo(Glu-His)], cyclo-arginyl-tyrosine [Cyclo(Arg-Tyr)], or cyclo-isoleucyl-serine [Cyclo(Ile-Ser)], or its corresponding salt in the composition according to the present invention is, in terms of a content per Brix, 0.2 ppm/Brix or more, preferably 1.0 ppm/Brix or more, more preferably 2.0 ppm/Brix or more, and $1.0 \times 10^4$ ppm/Brix or less, preferably $5.0 \times 10^3$ ppm/Brix or less, more preferably $1.0 \times 10^3$ ppm/Brix or less, and is typically 0.2 ppm/Brix to $1.0 \times 10^4$ ppm/Brix, preferably 1.0 ppm/Brix to $5.0 \times 10^3$ ppm/Brix, more preferably 2.0 ppm/Brix to $1.0 \times 10^3$ ppm/Brix.

In the case of using a synthesized product or a purified product as the cyclic dipeptide or salt thereof, the total amount of cyclo-glutamyl-histidine [Cyclo(Glu-His)], cyclo-arginyl-tyrosine [Cyclo(Arg-Tyr)], and cyclo-isoleucyl-serine [Cyclo(Ile-Ser)], and their corresponding salts in the composition according to the present invention is not particularly limited and is, for example, in terms of a content per Brix, 1.0 ppm/Brix or more, preferably 5.0 ppm/Brix or more, more preferably 10 ppm/Brix or more, and $1.0 \times 10^4$ ppm/Brix or less, preferably $5.0 \times 10^3$ ppm/Brix or less, more preferably $1.0 \times 10^3$ ppm/Brix or less, and is typically 1.0 ppm/Brix to $1.0 \times 10^4$ ppm/Brix, preferably 5.0 ppm/Brix to $1.0 \times 10^4$ ppm/Brix, more preferably 5.0 ppm/Brix to $5.0 \times 10^3$ ppm/Brix, still more preferably 10 ppm/Brix to $1.0 \times 10^3$ ppm/Brix. In the case of using a synthesized product or a purified product as the cyclic dipeptide or salt thereof, the individual content of cyclo-glutamyl-histidine [Cyclo(Glu-His)], cyclo-arginyl-tyrosine [Cyclo(Arg-Tyr)], or cyclo-isoleucyl-serine [Cyclo(Ile-Ser)], or each corresponding salt in the composition according to the present invention is, in terms of a content per Brix, 0.2 ppm/Brix or more, preferably 1.0 ppm/Brix or more, more preferably 2.0 ppm/Brix or more, and $1.0 \times 10^4$ ppm/Brix or less, preferably $5.0 \times 10^3$ ppm/Brix or less, more preferably $1.0 \times 10^3$ ppm/Brix or less, and is typically 0.2 ppm/Brix to $1.0 \times 10^4$ ppm/Brix, preferably 1.0 ppm/Brix to $5.0 \times 10^3$ ppm/Brix, more preferably 2.0 ppm/Brix to $1.0 \times 10^3$ ppm/Brix.

In the present invention, the content of a cyclic dipeptide or a salt thereof can be indicated by an amount per Brix as described above. "ppm" used in the present specification refers to ppm in weight / volume (w/v), unless otherwise specified, and 1.0 ppm/Brix can be converted into 0.1 mg/mL or 0.01% by weight when the specific gravity of the solvent is 1.

The content of the cyclic dipeptide or salt thereof added to the composition according to the present invention can be measured in accordance with a known method. The content can be measured by using, for example, LC-MS/MS or a saccharimeter.

The composition according to the present invention may be prepared, for example, by mixing the amino acid or salt thereof and the cyclic dipeptide or salt thereof in a predetermined amount with raw materials of a known composition in accordance with a known production method for a composition, or may be prepared by adding the amino acid or salt thereof and the cyclic dipeptide or salt thereof to a known ready-made composition so as to reach the predetermined amount followed by dissolving and/or suspending. A known composition originally containing the amino acid or salt thereof or the cyclic dipeptide or salt thereof may be used, and it may be appropriately mixed for preparation as long as the ratio of the total amount of the amino acid or salt thereof to the total amount of the cyclic dipeptide or salt thereof according to the present invention falls within the predetermined range.

4-1. Mechanism of action

Angiotensin-converting enzyme is mainly present in the lung, vascular endothelial cells, and the renal proximal tubule and has the property of converting the substrate angiotensin I to angiotensin II serving as a pressor hormone. This angiotensin II acts as a potent pressor hormone and promotes vasoconstriction by binding to its receptor present in the vascular vessel. Also, the angiotensin-converting enzyme has an action of decomposing bradykinin having a vasodilating action. Thus, an effect of suppressing production of the pressor hormone angiotensin II and an effect of suppressing decomposition of bradykinin having a vasodilating action are exerted through inhibition of the angiotensin-converting enzyme activity. This can be expected to produce a blood pressure elevation-suppressing action or a blood pressure-lowering action. Accordingly, the inhibition of the angiotensin-converting enzyme is clinically effective for treatment or prevention of hypertension.

4-2. Other components

The composition according to the present invention can contain any additive or any component commonly used, for example, in addition to the raw material containing the specific amino acid or salt --hereof and the specific cyclic dipeptide or salt thereof. Examples of such additives and/or components include, but not limited to, physiologically active components including vitamins, minerals, nutrient components, and fragrances, and in addition, diluents, binders, emulsifiers, tonicity agents (isotonic agents), buffers, solubilizers, antiseptic agents, stabilizers, antioxidants, colorants, coagulants, and coating agents to be mixed in formation.

4-3. Application

The composition according to the present invention can strongly enhance an angiotensin-converting enzyme-inhibiting activity brought about by an amino acid alone, by adjusting the ratio of the total amount of the amino acid or salt thereof to the total amount of the cyclic dipeptide or salt thereof to within a specific range. The composition according to the present invention thereby exerts a blood pressure elevation-suppressing action or a blood pressure-lowering action and can be used for prevention or amelioration of hypertension or prevention or amelioration of various diseases caused by hypertension, such as stroke, heart disease, and renal failure. Thus, one embodiment of the present invention is the composition for use for the suppression of elevation of blood pressure, for lowering of blood pressure, for protection of renal functions, or for prevention or amelioration of stroke or heart disease.

Throughout the present specification, examples of the diseases caused by hypertension include, but not particularly limited to, stroke, heart disease, and renal failure. Examples of the stroke include cerebral infarction, cerebral hemorrhage, and subarachnoid hemorrhage. Examples of the heart disease include angina pectoris, myocardial infarction, cardiomegaly, and heart failure.

Examples of the composition according to the present invention include, but not limited to, drugs, drinks, foods, and cosmetics. Specific examples of the drink according to the present invention may include oolong tea drinks, tea drinks, green tea drinks, fruit juice drinks, vegetable juices, sports drinks, isotonic drinks, enhanced waters, mineral waters, near waters, and energy drinks. Examples of the food according to the present invention may include functional foods, dietary supplements, foods with nutrient function claims, foods for special dietary uses, foods for specified health uses, nutritional supplements, foods for diet treatment, health foods, and supplements. The food or drink according to the present invention may be prepared, for example, by mixing the cyclic dipeptide or salt thereof in a predetermined amount with raw materials of a known food or drink in accordance with a known production method for a food, or may be prepared by adding the cyclic dipeptide or salt thereof to a known ready-made food or drink so as to reach the predetermined amount.

The composition according to the present invention is applicable to any of therapeutic applications (medical applications) and non-therapeutic applications (non-medical applications). Specific examples thereof include use for drugs, quasi-drugs, and cosmetics, and include use for compositions which do not belong to drugs, quasi-drugs, or cosmetics under the Pharmaceutical Affairs Act and nevertheless explicitly or implicitly features an effect of suppressing elevation of blood pressure, an effect of slowing elevation of blood pressure, an effect of suppressing rapid elevation of blood pressure upon awakening, a preventing effect on hypertension, an ameliorating effect on hypertension-, an effect of protecting renal functions, or an effect of ameliorating renal functions.

In another aaspect, the present disclosure relates to the composition provided with a label indicating a function to be exerted through inhibition of angiotensin-converting enzyme. Examples of such an indication or functional indication include, but not particularly limited to, "expecting lowering of blood pressure", "suppressing elevation of blood pressure", "slowing elevation of blood pressure", "suppressing rapid elevation of blood pressure upon awakening", "preventing hypertension", "helping ameliorating hypertension", "protecting renal functions", and "ameliorating renal functions". In the present disclosure, a label with such indication or functional indication may be provided to the composition itself, or provided to a container or package for the composition.

The composition according to the present invention can be ingested by using an appropriate method in accordance with its form. The ingestion method is not particularly limited as long as the amino acid or salt thereof and the cyclic dipeptide or salt thereof contained in the composition according to the present invention can be transferred into circulating blood. Examples of the form may include, but not limited to, solid preparations for oral administration such as tablets, coated tablets, granules, powders, and capsules, liquid preparations for oral administration such as oral solutions and syrups, and preparations for parenteral administration such as injections, external preparations, suppositories, and transdermal preparations. Throughout the present specification, "ingestion" is used to include all forms such as ingestion, administration, and drinking.

The amount of application of the composition according to the present invention is not constant, and is appropriately set in accordance with its form, administration method, intended use, and the age, bodyweight, and symptoms of a patient or patient animal as a subject to ingest the composition. The effective amount of ingestion of the composition according to the present invention for a human is not constant, and, for example, in the case of a human with a body weight of 50 kg, is preferably 10 mg or more, more preferably 50 mg or more, in terms of the weight of the amino acid or salt there of according to the present invention per day. Also, in the case of a human with a body weight of 50 kg, it is preferably 1.0 mg or more, more preferably 5.0 mg or more, in terms of the weight of the cyclic dipeptide or salt thereof according to the present invention per day. Administration may be performed in a single administration or multiple administrations in a day, within a desired range of the amount of application. Any duration may be used for the administration. Here, the effective amount of ingestion of the amino acid or salt thereof or the cyclic dipeptide or salt thereof for a human according to the present invention refers to the total amount of ingestion of the amino acid or salt thereof or the cyclic dipeptide or salt thereof which provides an effective action on a human, and is not limited by the type of the amino acid or the cyclic dipeptide.

The subject to apply the composition according to the present invention is preferably a human, but may be a domestic animal such as cattle, a horse, and a goat, a pet animal such as a dog, a cat, and a rabbit, or a laboratory animal such as a mouse, a rat, a guinea pig, and a monkey. In the case of administration for a non-human animal, the amount of use per day for a mouse of approximately 20 g varies depending on conditions including the contents of the amino acid or salt thereof and the cyclic dipeptide or salt thereof in the composition, and the condition, body weight, sex, and age of a subject to be applied, and in common cases is suitably set to such an amount that allows ingestion of preferably 10 mg/kg or more, more preferably 50 mg/kg or more, as the total amount of the amino acid or salt thereof to be added. Also, it is suitably set to such an amount that allows ingestion of preferably 1.0 mg/kg or more, more preferably 5.0 mg/kg or more, as the total amount of the cyclic dipeptide or salt thereof to be added.

5. Use of composition containing amino acid and cyclic dipeptide for inhibiting angiotensin-converting enzyme

One aspect of the present disclosure is the use of a composition containing an amino acid or a salt thereof and a cyclic dipeptide or a salt thereof for inhibiting angiotensin-converting enzyme, wherein the amino acid is one or two or more members selected from the group consisting of glycine, alanine, valine, leucine, isoleucine, serine, threonine, aspartic acid, glutamic acid, asparagine, glutamine, arginine, lysine, cysteine, methionine, phenylalanine, tyrosine, tryptophan, histidine, proline, and hydroxyproline, the cyclic dipeptide is one or two or more members selected from the group consisting of cyclo-glutamyl-histidine [Cyclo(Glu-His)], cyclo-arginyl-tyrosine [Cyclo(Arg-Tyr)], and cyclo-isoleucyl-serine [Cyclo(Ile-Ser)], and the ratio of the total amount of the amino acid or salt thereof to the total amount of the cyclic dipeptide or salt thereof is 1:0.02 to 1:20.

Among the amino acids described above or salts thereof, one or two or more members selected from the group consisting of leucine, threonine, arginine, tyrosine, and tryptophan are preferred, and three or more members selected from these amino acids or salts thereof are more preferably contained.

The type and content of the amino acid or salt thereof, the type and content of the cyclic dipeptide or salt thereof, the range of the ratio of the total amount of the amino acid or salt thereof to the total amount of the cyclic dipeptide or salt thereof, etc. are as described above about the composition. Specific examples and amounts of other components that may be added are also as described above about the composition.

The use according to the present invention includes, but not limited to, for example, use for suppression of elevation of blood pressure, lowering of blood pressure, protection of renal functions, or prevention or amelioration of stroke, heart disease, or hypertension. The use is use for a human or a non-human animal, and may be therapeutic use or non-therapeutic use. Here, "non-therapeutic" is a concept excluding medical practice, that is, therapeutic practice for a human body through treatment.

6. Method for inhibiting angiotensin-convertin genzyme

One aspect of the present disclosure is a method for inhibiting angiotensin-converting enzyme using a composition containing an amino acid or a salt thereof and a cyclic dipeptide or a salt thereof, wherein the amino acid is one or two or more members selected from the group consisting of glycine, alanine, valine, leucine, isoleucine, serine, threonine, aspartic acid, glutamic acid, asparagine, glutamine, arginine, lysine, cysteine, methionine, phenylalanine, tyrosine, tryptophan, histidine, proline, and hydroxyproline, the cyclic dipeptide is one or two or more members selected from the group consisting of cyclo-glutamyl-histidine [Cyclo(Glu-His)], cyclo-arginyl-tyrosine [Cyclo(Arg-Tyr)], and cyclo-isoleucyl-serine [Cyclo(Ile-Ser)], and the ratio of the total amount of the amino acid or salt thereof to the total amount of the cyclic dipeptide or salt thereof is 1:0.02 to 1:20.

Among the amino acids described above or salts thereof, one or two or more members selected from the group consisting of leucine, threonine, arginine, tyrosine, and tryptophan are preferred, and three or more members selected from these amino acids or salts thereof are more preferably contained.

The type and content of the amino acid or salt thereof, the type and content of the cyclic dipeptide or salt thereof, the range of the ratio of the total amount of the amino acid or salt thereof to the total amount of the cyclic dipeptide or salt thereof, etc. are as described above about the composition. Specific examples and amounts of other components that may be added are also as described above about the composition.

In the method, an individual in need of inhibition of angiotensin-converting enzyme refers to the above subject for application of the composition according to the present invention. Throughout the present specification, a therapeutically effective amount refers to an amount at which angiotensin-converting enzyme in the individual administered with the composition according to the present invention is inhibited compared to the case of an individual without administration. A specific effective amount is not constant, and is set appropriately in accordance with form of administration, an administration method, intended use, and the age, body weight, and symptoms of an individual.

In the method according to the present invention, the specific amino acid or salt thereof or the specific cyclic dipeptide or salt thereof may be administered as it is, or as a composition containing the specific amino acid or salt thereof or the specific cyclic dipeptide or salt thereof so as to reach the therapeutically effective amount.

The method according to the present invention enables inhibition of angiotensin-converting enzyme without any side effect.

EXAMPLES

The present invention will now be described with reference to Examples, but the scope of the present invention is not limited thereby. Those skilled in the art could use the method according to the present invention with various variations or modifications, which are also included in the scope of the present invention.

Example 1. Enhancing effect of cyclic dipeptide on angiotensin-converting enzyme-inhibiting action of amino acid

The enhancing effect of a cyclic dipeptide on the angiotensin-converting enzyme-inhibiting action of an amino acid was evaluated. Specifically, as for the inhibiting effects of amino acids and cyclic dipeptides, angiotensin I-ccnverting enzyme-inhibiting activities were measured by using ACE Kit-WST (Dojindo Laboratories) according to the instruction manual attached to the kit. The amino acids were studied at a concentration of 1000 μM, 3000 μM, or 5000 μM, and the cyclic dipeptides were studied at a concentration of 20 μM, 50 μM, 100 μM, 250 μM, or 500 μM. Their-inhibiting activities were evaluated as the residual activity of angiotensin-converting enzyme calculated according to expression 1 given below. A control supplemented with distilled water instead of the aqueous cyclic dipeptide solution was used. Chemically synthesized samples were used as the cyclic dipeptides.

(Expression 1)

Residual activity (%) = (Absorbance at 450 nm from the cyclic dipeptide-added group / Absorbance from 450 nm from the control) × 100

The evaluation results of various cyclic dipeptides are shown in Tables 1 to 4 below.

[Table 1]

| Amino acid | Concentration (μM) | Cyclic dipeptide | Concentration (μM) | Residual activity % | Standard deviation |
|---|---|---|---|---|---|
| Leucine (Leu) | 1000 | | | 83.2 | 2.6 |
| | | Cyclo (Glu-His) | 50 | 82.9 | 1.4 |
| | | | 250 | 79.2 | 1.3 |
| | | Cyclo (Arg-Tyr) | 20 | 80.0 | 0.8 |
| | | | 100 | 78.2 | 0.7 |
| | | | 500 | 68.7 | 1.7 |
| | | Cyclo (Ile-Ser) | 20 | 82.7 | 2.4 |
| | | | 100 | 73.9 | 3.4 |
| | | | 500 | 61.0 | 2.6 |
| | 5000 | | | 43.3 | 0.0 |

[Table 2]

| Amino acid | Concentration (μM) | Cyclic dipeptide | Concentration (μM) | Residual activity % | Standard deviation |
|---|---|---|---|---|---|
| Threonin e (Thr) | 1000 | | | 86.6 | 0.0 |
| | | Cyclo (Glu-His) | 20 | 83.6 | 0.2 |
| | | | 100 | 83.0 | 2.9 |
| | | | 500 | 65.4 | 2.6 |
| | | Cyclo (Arg-Tyr) | 20 | 81.3 | 0.9 |
| | | | 100 | 85.9 | 6.5 |
| | | | 500 | 79.8 | 0.0 |
| | 5000 | | | 69.0 | 1.4 |

[Table 3]

| Amino acid | Concentration (μM) | Cyclic dipeptide | Concentration (μM) | Residual activity % | Standard deviation |
|---|---|---|---|---|---|
| Arginine (Arg) | 1000 | | | 84.8 | 7.2 |
| | | Cyclo (Glu-His) | 20 | 81.1 | 2.3 |
| | | | 100 | 80.8 | 1.5 |
| | | | 500 | 78.2 | 0.7 |
| | | Cyclo (Arg- Tyr) | 20 | 83.4 | 3.8 |
| | | | 100 | 67.4 | 8.7 |
| | | | 500 | 66.5 | 0.9 |
| | | Cyclo (Ile-Ser) | 100 | 83.4 | 1.3 |
| | | | 500 | 78.8 | 1.9 |
| | 5000 | | | 60.2 | 1.2 |

[Table 4]

| Amino acid | Concentration (μM) | Cyclic dipeptide | Concentration (μM) | Residual activity % | Standard deviation |
|---|---|---|---|---|---|
| Tyrosine (Tyr) | 1000 | | | 94.0 | 0.0 |
| | | Cyclo (Glu-HiS) | 100 | 92.8 | 1.5 |
| | | | 500 | 87.6 | 2.6 |
| | | Cyclo (Arg- Tyr) | 100 | 93.8 | 3.2 |
| | | | 500 | 81.3 | 0.2 |
| | | Cyclo (Ile-Ser) | 100 | 93.5 | 2.3 |
| | | | 500 | 72.9 | 2.9 |
| | 3000 | | | 90.6 | 2.2 |

The results of Tables 1 to 4 suggested that the angiotensin-converting enzyme-inhibiting action brought about by the amino acid is enhanced by addition of the specific cyclic dipeptide (cyclo-glutamyl-histidine [Cyclo (Glu-His)], cyclo-arginyl-tyrosine [Cyclo(Arg-Tyr)], and cyclo-isoleucyl-serine [Cyclo(Ile-Ser)]) in a given amount to the amino acid.

INDUSTRIAL APPLICABILITY

The present invention relates to a composition containing an amino acid or a salt thereof and a cyclic dipeptide or a salt thereof at a given ratio. This can enhance an angiotensin-converting enzyme-inhibiting activity brought about by the amino acid alone. The amino acid contained in the composition according to the present invention is a naturally derived component, and the cyclic dipeptide is also contained in a heat-treated product of a plant-derived peptide, or the like. Thus, these components are highly safe, and the composition according to the present invention has a very low risk of manifesting serious side effects. Cyclic dipeptides have high lipid solubility and are efficiently absorbed from the gastrointestinal tract. Thus, the present invention provides a novel means to contribute to suppression of elevation of blood pressure, etc. , and thus has high industrial applicability.

**Claims**

1. A composition comprising an amino acid or a salt thereof and a cyclic dipeptide or a salt thereof, wherein
the amino acid is one or two or more members selected from the group consisting of glycine, alanine, valine, leucine, isoleucine, serine, threonine, aspartic acid, glutamic acid, asparagine, glutamine, arginine, lysine, cysteine, methionine, phenylalanine, tyrosine, tryptophan, histidine, proline, and hydroxyproline,
the cyclic dipeptide is one or two or more members selected from the group consisting of cyclo-glutamyl-histidine [Cyclo(Glu-His)], cyclo-arginyl-tyrosine [Cyclo(Arg-Tyr)], and cyclo-isoleucyl-serine [Cyclo(Ile-Ser)], and
the ratio of the total amount of the amino acid or salt thereof to the total amount of the cyclic dipeptide or salt thereof is 1:0.02 to 1:20.

2. The composition according to claim 1, wherein
the amino acid is leucine, and
the ratio of the total amount of the amino acid or salt thereof to the total amount of the cyclic dipeptide or salt thereof is 1:0.02 to 1:1.5,

3. The composition according to claim 1, wherein
the amino acid is threonine, and
the ratio of the total amount of the amino acid or salt thereof to the total amount of the cyclic dipeptide or salt thereof is 1:0.02 to 1:1.5.

4. The composition according to claim 1, wherein
the amino acid is arginine, and
the ratio of the total amount of the amino acid or salt thereof to the total amount of the cyclic dipeptide or salt thereof is 1:0.02 to 1:1.5.

5. The composition according to claim 1, wherein
the amino acid is tyrosine, and
the ratio of the total amount of the amino acid or salt thereof to the total amount of the cyclic dipeptide or salt thereof is 1:0.10 to 1:1.5.

6. The composition according to claim 1, wherein
the amino acid is tryptophan, and
the ratio of the total amount of the amino acid or salt thereof to the total amount of the cyclic dipeptide or salt thereof is 1:0.02 to 1:1.5.

7. The composition according to any one of claims 1 to 6, wherein the total amount of the cyclic dipeptide or salt thereof is 1.0 ppm/Brix to $1.0 \times 10^4$ ppm/Brix.

8. The composition according to any one of claims 1 to 6, wherein the total amount of the cyclic dipeptide or salt thereof is 5.0 ppm/Brix to $1.0 \times 10^4$ ppm/Brix.

9. The composition according to any one of claims 1 to 8, wherein the amino acid or salt thereof is obtained from a soybean peptide, a tea peptide, or a malt peptide.

10. The composition according to any one of claims 1 to 9, wherein the cyclic dipeptide or salt thereof is obtained from a soybean peptide, a tea peptide, or a malt peptide.

11. The composition according to any one of claims 1 to 10, wherein the composition has an angiotensin-converting enzyme-inhibiting action.

12. The composition according to any one of claims 1 to 11, wherein the composition is for use in suppression of elevation of blood pressure, for lowering of blood pressure, for protection of renal functions, or for prevention or amelioration of stroke or heart disease.

13. A composition comprising an amino acid or a salt thereof and a cyclic dipeptide or a salt thereof for use in inhibiting angiotensin-converting enzyme, wherein
the amino acid is one or two or more members selected from the group consisting of glycine, alanine, valine, leucine, isoleucine, serine, threonine, aspartic acid, glutamic acid, asparagine, glutamine, arginine, lysine, cysteine, methionine, phenylalanine, tyrosine, tryptophan, histidine, proline, and hydroxyproline,
the cyclic dipeptide is one or two or more members selected from the group consisting of cyclo-glutamyl-histidine [Cyclo(Glu-His)], cyclo-arginyl-tyrosine [Cyclo(Arg-Tyr)], and cyclo-isoleucyl-serine [Cyclo(Ile-Ser)], and
the ratio of the total amount of the amino acid or salt thereof to the total amount of the cyclic dipeptide or salt thereof is 1:0.02 to 1:20.

14. The composition for use according to claim 13, wherein the amino acid includes one or two or more members selected from the group consisting of leucine, threonine, arginine, tyrosine, and tryptophan.

**Patentansprüche**

1. Eine Zusammensetzung, umfassend eine Aminosäure oder ein Salz davon und ein cyclisches Dipeptid oder ein Salz davon, wobei
es sich bei der Aminosäure um ein oder zwei oder mehrere Elemente handelt, ausgewählt aus der Gruppe bestehend aus Glycin, Alanin, Valin, Leucin, Isoleucin, Serin, Threonin, Aspartamsäure, Glutaminsäure, Asparagin, Glutamin, Arginin, Lysin, Cystein, Methionin, Phenylalanin, Tyrosin, Tryptophan, Histidin, Prolin und Hydroxyprolin,
es sich bei dem cyclischen Dipeptid um ein oder zwei oder mehrere Elemente handelt, ausgewählt aus der Gruppe bestehend aus Cyclo-glutamyl-histidin [Cyclo(Glu-His)], Cyclo-arginyl-tyrosin [Cyclo(Arg-Tyr)] und Cyclo-isoleucyl-serin [Cyclo(Ile-Ser)], und
das Verhältnis der Gesamtmenge der Aminosäure oder des Salzes davon zu der Gesamtmenge des cyclischen Dipeptids oder des Salzes davon 1:0,02 bis 1:20 beträgt.

2. Die Zusammensetzung nach Anspruch 1, wobei
es sich bei der Aminosäure um Leucin handelt, und
das Verhältnis der Gesamtmenge der Aminosäure oder des Salzes davon zu der Gesamtmenge des cyclischen Dipeptids oder des Salzes davon 1:0,02 bis 1:1,5 beträgt.

3. Die Zusammensetzung nach Anspruch 1, wobei
es sich bei der Aminosäure um Threonin handelt, und
das Verhältnis der Gesamtmenge der Aminosäure oder des Salzes davon zu der Gesamtmenge des cyclischen Dipeptids oder des Salzes davon 1:0,02 bis 1:1,5 beträgt.

4. Die Zusammensetzung nach Anspruch 1, wobei
es sich bei der Aminosäure um Arginin handelt, und
das Verhältnis der Gesamtmenge der Aminosäure oder des Salzes davon zu der Gesamtmenge des cyclischen Dipeptids oder des Salzes davon 1:0,02 bis 1:1,5 beträgt.

5. Die Zusammensetzung nach Anspruch 1, wobei
es sich bei der Aminosäure um Tyrosin handelt, und
das Verhältnis der Gesamtmenge der Aminosäure oder des Salzes davon zu der Gesamtmenge des cyclischen Dipeptids oder des Salzes davon 1:0,10 bis 1:1,5 beträgt.

6. Die Zusammensetzung nach Anspruch 1, wobei
es sich bei der Aminosäure um Tryptophan handelt, und

das Verhältnis der Gesamtmenge der Aminosäure oder des Salzes davon zu der Gesamtmenge des cyclischen Dipeptids oder des Salzes davon 1:0,02 bis 1:1,5 beträgt.

7. Die Zusammensetzung nach einem der Ansprüche 1 bis 6, wobei die Gesamtmenge des cyclischen Dipeptids oder des Salzes davon 1,0 ppm/Brix bis $1,0 \times 10^4$ ppm/Brix beträgt.

8. Die Zusammensetzung nach einem der Ansprüche 1 bis 6, wobei die Gesamtmenge des cyclischen Dipeptids oder des Salzes davon 5,0 ppm/Brix bis $1,0 \times 10^4$ ppm/Brix beträgt.

9. Die Zusammensetzung nach einem der Ansprüche 1 bis 8, wobei die Aminosäure oder das Salz davon aus einem Sojabohnenpeptid, einem Teepeptid oder einem Malzpeptid erhalten wird.

10. Die Zusammensetzung nach einem der Ansprüche 1 bis 9, wobei das cyclische Dipeptid oder das Salz davon aus einem Sojabohnenpeptid, einem Teepeptid oder einem Malzpeptid erhalten wird.

11. Die Zusammensetzung nach einem der Ansprüche 1 bis 10, wobei die Zusammensetzung eine inhibierende Wirkung auf Angiotensin-konvertierendes Enzym aufweist.

12. Die Zusammensetzung nach einem der Ansprüche 1 bis 11, wobei die Zusammensetzung zur Verwendung bei der Unterdrückung einer Erhöhung des Blutdrucks, zur Senkung des Blutdrucks, zum Schutz der Nierenfunktionen oder zur Vorbeugung oder Linderung von Schlaganfall oder einer Herzerkrankung bestimmt ist.

13. Eine Zusammensetzung, umfassend eine Aminosäure oder ein Salz davon und ein cyclisches Dipeptid oder ein Salz davon zur Verwendung bei der Inhibition des Angiotensinkonvertierenden Enzyms, wobei
es sich bei der Aminosäure um ein oder zwei oder mehrere Elemente handelt, ausgewählt aus der Gruppe bestehend aus Glycin, Alanin, Valin, Leucin, Isoleucin, Serin, Threonin, Aspartamsäure, Glutaminsäure, Asparagin, Glutamin, Arginin, Lysin, Cystein, Methionin, Phenylalanin, Tyrosin, Tryptophan, Histidin, Prolin und Hydroxyprolin,
es sich bei dem cyclischen Dipeptid um ein oder zwei oder mehrere Elemente handelt, ausgewählt aus der Gruppe bestehend aus Cyclo-glutamyl-histidin [Cyclo(Glu-His)], Cyclo-arginyl-tyrosin [Cyclo(Arg-Tyr)] und Cyclo-isoleucyl-serin [Cyclo(Ile-Ser)], und
das Verhältnis der Gesamtmenge der Aminosäure oder des Salzes davon zu der Gesamtmenge des cyclischen Dipeptids oder des Salzes davon 1:0,02 bis 1:20 beträgt.

14. Die Zusammensetzung zur Verwendung nach Anspruch 13, wobei die Aminosäure ein oder zwei oder mehrere Elemente, ausgewählt aus der Gruppe bestehend aus Leucin, Threonin, Arginin, Tyrosin und Tryptophan, einschließt.

## Revendications

1. Composition comprenant un acide aminé ou un sel de celui-ci et un dipeptide cyclique ou un sel de celui-ci, dans laquelle
l'acide aminé est un ou deux ou plusieurs membres choisis dans le groupe consistant en la glycine, l'alanine, la valine, la leucine, l'isoleucine, la sérine, la thréonine, l'acide aspartique, l'acide glutamique, l'asparagine, la glutamine, l'arginine, la lysine, la cystéine, la méthionine, la phénylalanine, la tyrosine, le tryptophane, l'histidine, la proline et l'hydroxyproline,
le dipeptide cyclique est un ou deux ou plusieurs membres choisis dans le groupe consistant en la cyclo-glutamyl-histidine [Cyclo(Glu-His)], la cyclo-arginyl-tyrosine [Cyclo(Arg-Tyr)] et la cyclo-isoleucyl-sérine [Cyclo(Ile-Ser)], et
le rapport de la quantité totale de l'acide aminé ou de son sel à la quantité totale du dipeptide cyclique ou de son sel est 1:0,02 à 1:20.

2. Composition selon la revendication 1, dans laquelle
l'acide aminé est la leucine, et
le rapport de la quantité totale de l'acide aminé ou de son sel à la quantité totale du dipeptide cyclique ou de son sel est 1:0,02 à 1:1,5.

3. Composition selon la revendication 1, dans laquelle
l'acide aminé est la thréonine, et

le rapport de la quantité totale de l'acide aminé ou de son sel à la quantité totale du dipeptide cyclique ou de son sel est 1:0,02 à 1:1,5.

4. Composition selon la revendication 1, dans laquelle
l'acide aminé est l'arginine, et
le rapport de la quantité totale de l'acide aminé ou de son sel à la quantité totale du dipeptide cyclique ou de son sel est 1:0,02 à 1:1,5.

5. Composition selon la revendication 1, dans laquelle
l'acide aminé est la tyrosine, et
le rapport de la quantité totale de l'acide aminé ou de son sel à la quantité totale du dipeptide cyclique ou de son sel est 1:0,10 à 1:1,5.

6. Composition selon la revendication 1, dans laquelle
l'acide aminé est le tryptophane, et
le rapport de la quantité totale de l'acide aminé ou de son sel à la quantité totale du dipeptide cyclique ou de son sel est 1:0,02 à 1:1,5.

7. Composition selon l'une quelconque des revendications 1 à 6, dans laquelle la quantité totale du dipeptide cyclique ou de son sel est 1,0 ppm/Brix à $1,0 \times 10^4$ ppm/Brix.

8. Composition selon l'une quelconque des revendications 1 à 6, dans laquelle la quantité totale du dipeptide cyclique ou de son sel est 5,0 ppm/Brix à $1,0 \times 10^4$ ppm/Brix.

9. Composition selon l'une quelconque des revendications 1 à 8, dans laquelle l'acide aminé ou son sel est obtenu à partir d'un peptide de soja, d'un peptide de thé ou d'un peptide de malt.

10. Composition selon l'une quelconque des revendications 1 à 9, dans laquelle le dipeptide cyclique ou son sel est obtenu à partir d'un peptide de soja, d'un peptide de thé ou d'un peptide de malt.

11. Composition selon l'une quelconque des revendications 1 à 10, dans laquelle la composition a une action inhibant l'enzyme de conversion de l'angiotensine.

12. Composition selon l'une quelconque des revendications 1 à 11, dans laquelle la composition est destinée à être utilisée dans la suppression de l'élévation de la pression artérielle, pour abaisser la pression artérielle, pour la protection des fonctions rénales, ou pour la prévention ou l'amélioration des accidents vasculaires cérébraux ou des maladies cardiaques.

13. Composition comprenant un acide aminé ou un sel de celui-ci et un dipeptide cyclique ou un sel de celui-ci destinée à être utilisée dans l'inhibition de l'enzyme de conversion de l'angiotensine, dans laquelle
l'acide aminé est un ou deux ou plusieurs membres choisis dans le groupe consistant en la glycine, l'alanine, la valine, la leucine, l'isoleucine, la sérine, la thréonine, l'acide aspartique, l'acide glutamique, l'asparagine, la glutamine, l'arginine, la lysine, la cystéine, la méthionine, la phénylalanine, la tyrosine, le tryptophane, l'histidine, la proline et l'hydroxyproline,
le dipeptide cyclique est un ou deux ou plusieurs membres choisis dans le groupe consistant en la cyclo-glutamyl-histidine [Cyclo(Glu-His)], la cyclo-arginyl-tyrosine [Cyclo(Arg-Tyr)] et la cyclo-isoleucyl-sérine [Cyclo(Ile-Ser)], et
le rapport de la quantité totale de l'acide aminé ou de son sel à la quantité totale du dipeptide cyclique ou de son sel est 1:0,02 à 1:20.

14. Composition destinée à être utilisée selon la revendication 13, dans laquelle l'acide aminé inclut un ou deux ou plusieurs membres choisis dans le groupe consistant en la leucine, la thréonine, l'arginine, la tyrosine et le tryptophane.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011102327 A
- JP 2003252896 A
- WO 2014200000 A

**Non-patent literature cited in the description**

- *Clin Exp Pharmacol Physiol.,* 2010, vol. 37 (1), 62-68
- *J. Brew. Soc. Japan.,* 2001, vol. 96 (3), 199-206
- *J. Pharmacol,* 1998, vol. 50, 167-172
- *Journal of Peptide Science,* 2004, vol. 10, 737-737